# EUROPEAN PATENT SPECIFICATION

(11) **EP 3 858 794 B1**
(45) Date of publication and mention of the grant of the patent: **21.10.2026**
(21) Application number: 20154781.7
(22) Date of filing: 31.01.2020
(51) Int. Cl.: C03B 27/04, C03B 29/02, C03B 29/08, G01N 21/896, G01J 5/00, G01J 5/48, G01J 5/80, G01N 33/38

(54) **METHOD FOR TEMPERING A GLASS SHEET**
VERFAHREN ZUR TEMPERIERUNG EINER GLASSCHEIBE
PROCÉDÉ DE TREMPE DE PLAQUE DE VERRE

(43) Date of publication of application: 04.08.2021
(73) Proprietor: Glaston Finland Oy, 33730 Tampere (FI)
(72) Inventor: Aronen, Antti, 33900 Tampere (FI); Rantala, Mikko, 38510 Sastamala (FI)
(74) Representative: LEITZINGER OY

(56) References cited:
- WO-A1-2017/176634
- US-A1- 2003 076 487
- US-A1- 2019 376 909

## Description

### Field of the invention

The object of the invention is a method for tempering a glass sheet.

### Background of the invention

The use of a tempering furnace is known for tempering glass sheets, wherein glass sheets are heated to a tempering temperature. The glass sheets move in the tempering furnace in one direction or back and forth on rotating ceramic rollers, on a so-called roller conveyor. A furnace equipped with a roller conveyor is often referred to as a roller furnace and the typical temperature thereof is, for example, from 680 to 700 °C. From the tempering furnace, the glass sheets move sequentially, side by side or in various glass loadings of one or more glass sheets along the roller conveyor to a tempering-cooling unit at the back of the furnace, where the temper cooling is carried out with air jets. Usually, the temperature of the air used for cooling is approximately the same as the air temperature outside or within the plant. A blower or a compressor is used for feeding the cooling air.

Instead of a roller furnace, it is possible to use a furnace based on air jet levitation, often also referred to as an air jet levitation furnace. In solutions based on the air jet levitation technique, the glass sheet floats supported by a thin air cushion and touches the conveyor line rollers or other conveying members only at one of its lateral edges. An equivalent air jet levitation technique can be applied in the tempering cooling unit attached to the back of the furnace.

The glass sheet entering the tempering process is so-called float glass, wherein the internal stress profile is low enough to allow clean cutting of the glass sheet. The compressive stress at the float glass surface is typically 1 to 4 MPa. As the glass sheet bends, tensile stress is formed at its convex surface, which the float glass surface withstands on average up to about 30 MPa. This limit is reached with a relatively small load, and as it is surpassed, the float glass will easily break. The aim of the tempering process is a sufficient increase in the strength of the float glass sheet.

In addition to strength, another desired property of the tempered glass sheet is safety when the glass sheet breaks. An untempered glass sheet will break into large pieces that pose a risk of cuts. A tempered glass sheet will instead break into almost harmless particles.

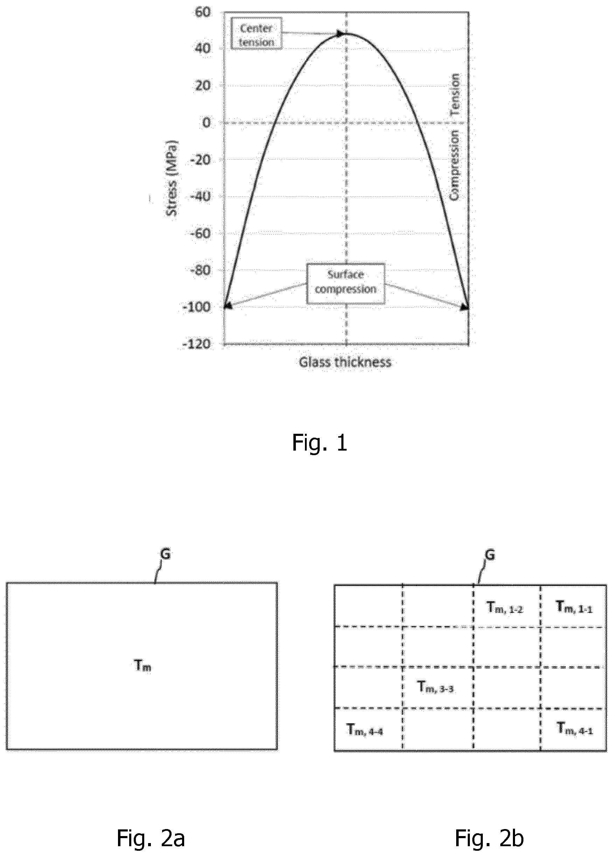

The compressive stress (hardening, i.e. tempering level) formed at the surface of the glass sheet during tempering is dependent on the thickness direction temperature profile of the glass sheet as the glass cools through a temperature zone characteristic of a glass sheet (about 600→500 °C). Typically, the temperature difference between the glass surface and the center thickness in tempering cooling is approximately 100 °C. The thinner the glass sheet, the more cooling power is required to reach the same above-mentioned temperature difference. During tempering, a permanent thickness direction stress distribution, often in the shape of a parabola, is formed. The internal permanent stresses within the glass sheet are referred to as residual stresses. Figure 1 shows the stress distribution as a function of the glass thickness. As can be seen from Figure 1, at the glass surfaces, i.e. at the ends of the parabola, the stress is compressive stress. In the center of the glass, i.e. in the center of the parabola, the stress is tensile stress. For example, for a glass sheet with a thickness of 4 mm, surface compression of at least about 100 MPa is sought during the tempering, whereby the tensile stress in the center of the glass is about 46 MPa. A 4 mm glass sheet with the center layer tensile stress in this range will upon fragmentation break into particles in a way that fulfils the requirements of safety glass standards. The fragmentation pattern of tempered glass, i.e. the density of particles in an area of glass, is dependent particularly on the amount of tensile stress within the glass. The relationship between density of tempered glass particles and residual stress, i.e. the tempering level, has been studied widely in literature. In the figures of the publication Akeyoshi, K. and Kanai, E., Mechanical properties of tempered glass, Proceedings of the VII:th Int. Glass Cong., Paper No. 80, 1965., the particle count in a glass area sized 50 x 50 mm is presented with relation to the measured glass tempering level. It can be concluded from the figures in the publication that the residual stress required to achieve the same particle count increases as the glass sheet becomes thinner. In the publication Lee, H., Cho, S., Yoon, K. and Lee, J., Glass thickness and fragmentation behavior in stressed glasses, New Journal of Glass and Ceramics, pp. 116-212, 2012, it was discovered that the density of the particles decreases drastically when the tensile layer starts at a greater depth than 20 % of the glass thickness. Thus, also the shape of the stress distribution affects the fragmentation of the glass.

As the tempered glass sheet is bent, tensile stress will start to form at its convex surface only when the compressive stress at the glass sheet surface formed during the tempering process is cancelled out. For example, the above-mentioned tempered glass sheet with a thickness of 4 millimeters withstands bending approximately ((100 MPa +30 MPa) / 30 MPa =) 4,3 times more than an untempered glass sheet. In general, tempered glass is considered to be 3 to 4 times stronger than a float glass sheet.

Many standards have been established to define tempered glass that qualifies as safety glass. Tempered glass that is acceptable as safety glass must, according to the standard EN 12150-1, fragment when broken with a pointed steel tool in such a way that each area of the glass sized 50 x 50 mm, not including the site of breakage and the edge strips, must have a particle count according to the following table. In the particle count, the number of particles entirely inside the 50 x 50 mm area is counted as one and the number of particles partially within the above-mentioned area is counted as half.

**Table 1: The lowest allowed particle count in broken safety glass according to the standard EN 12150-1.**

| Glass type | Nominal thickness in mm | Minimum particle count inside 50 × 50 mm area |
|---|---|---|
| Float and drawn sheet | 3 | 15 |
| Float and drawn sheet | 4 to 12 | 40 |
| Float and drawn sheet | 15 to 19 | 30 |
| Patterned | 4 to 10 | 30 |

A known problem is that for tempered glass, it is impossible to see whether the glass will break in a safe manner and whether the fragmentation of the glass will fulfil the requirements of the safety glass standard without breaking it. Breaking large and thick production glasses in particular is expensive, thus in general small test glasses intended specifically for testing are used for testing. In such cases, a test glass of equal thickness is added to the first glass loading of the first production series, which glass is then broken on purpose and examined whether its fragmentation fulfils the requirements of the safety glass standard. If so, all glasses in the glass loading are found to be tempered safety glass. The subsequent glass loadings are then tempered using the same tempering recipe, i.e. instructions, assuming it will temper them to safety glass. However, the glass sheets and different subsections of the glass sheets within a glass loading are typically not heated to the exact same tempering temperature as the load on the heating elements varies according to the amount of glass moving past them, and in an area with a smaller load, the glass sheets are heated more. Some of the heating elements in the furnace may also have broken, slowing down the heating of the glass mass moving past them. In addition, measures taken to adjust the heating to ensure uniform heating of the glass may fail and increase temperature differences in the glass. In general, a glass loading that is not full will be heated at a faster rate than a full one, and large glasses are heated at a slower rate than small ones. Thus, confirming that the test glass is safety glass does not ensure that all glasses in a production series are safety glass. To ensure it, a notably higher particle count than that set out in the standard is required for the test glass. Even this measure does not exclude the possibility of having tempered glass breaking in a dangerous manner among the production glasses. Even successful heating will not guarantee it. The use of too low a blowing pressure or too long a blowing distance, due to operator error, for example, may result in producing glass not qualifying as safety glass according to the safety glass standard. In a typical quality control protocol, a new test glass must be tempered each time the glass quality and thickness changes, which is common in the production of many operators in the industry. Test glasses thus also present a clear added cost.

There are some commercial devices for measuring the residual stress of tempered glass. A measuring device with the trade name GASP measures compressive stress at the glass surface, and a measuring device with the trade name SCALP measures the internal residual stress profile of the glass. Both devices are based on the different refraction of laser light depending on the residual stresses. However, both devices are only suitable for measurements in a laboratory, as measuring with them is relatively slow for monitoring the residual stresses in production glasses. In both measuring devices, GASP, SCALP, a small amount of liquid is dropped onto the surface of the glass, in GASP, a special oil and in SCALP, a special alcohol. The measuring device to be used is positioned on top of the liquid and it is adjusted, after which the stress can be read either directly or using auxiliary tables. The accuracy of the devices is satisfactory, i.e. the absolute stress can be obtained with a margin of error of about 5 %. However, measuring using the devices is difficult to automate, and the use of the device is not suitable for measurements where stress is to be measured from moving glass. In patent application CN 107677402A, a method for utilizing devices known in the prior art has been disclosed.

Measuring the stress of tempered glass has also been discussed in patent application US 2003/0076487. The aim therein is to measure the residual stress of the glass based on the behavior of polarized light in the glass. In this method, touching of the glass is not required.

The method of patent application US 2017/0221198A facilitates the quality control of tempered glass by automating the analysis of the particle pattern of tempered glass. In the method, the broken tempered glass is illuminated and photographed, and the relevant details of the particle pattern are analyzed from the photo using an image processing program.

### Summary of invention

The object of the present invention is to solve the above-mentioned problems and provide a method for tempering a glass sheet with which method the quality of glass sheets, particularly the tempering level of glass sheets, can be monitored during production and thereby reduce or prevent potentially low quality glass sheets with a tempering level not sufficient in quality from entering the market.

This object is achieved with the method described in the accompanying independent claim. The preferred embodiments of the invention are described in the dependent claims.

The invention is based on determining the fragmentation pattern of the glass sheet based on the residual stress profile of the glass sheet. Once the residual stress profile of the glass sheet, particularly the amount of the internal tensile stress of the glass sheet, is known, the particle count of the glass sheet according to the standard EN12150-1 can be determined.

### Brief description of drawings

The invention will now be described in more detail with reference to the accompanying drawings, in which:
- Figure 1: shows the stress distribution as a function of the glass thickness,
- Figure 2a: shows a measuring method for measuring the temperature of the glass sheet,
- Figure 2b: shows a measuring method for measuring the temperature of the glass sheet,
- Figure 3: shows a tempering apparatus according to a first embodiment of the present invention,
- Figure 4: shows a tempering apparatus according to another embodiment of the present invention,
- Figure 5: shows a block diagram of a tempering apparatus according an embodiment of the invention,
- Figure 6: shows a flow chart of a method according to an embodiment of the invention.

### Description of other embodiments of the invention

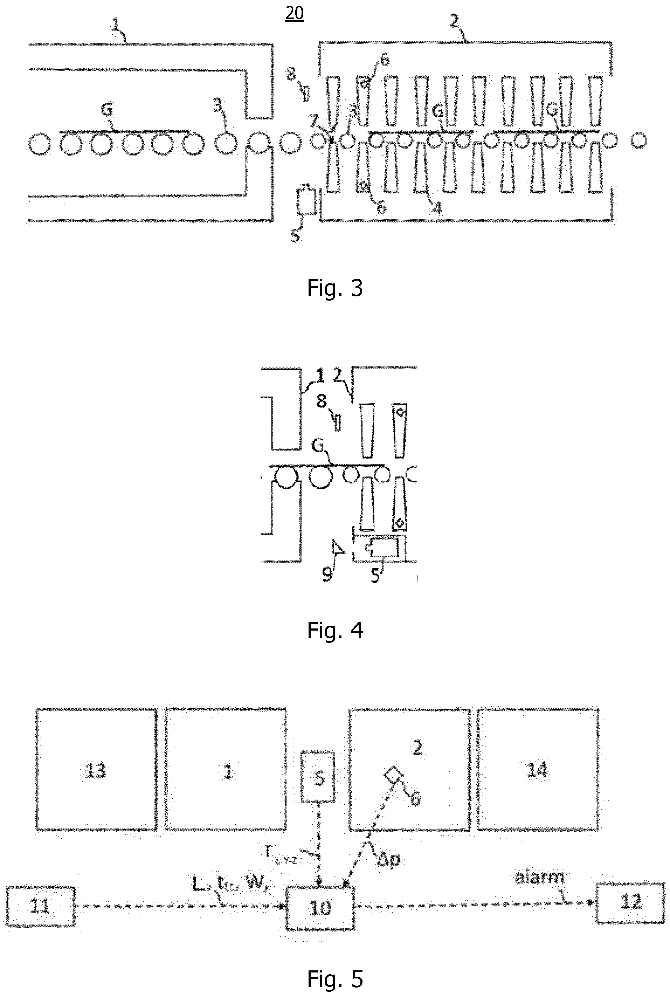

Figure 3 shows a tempering apparatus which can be used to implement said claimed method.

The tempering apparatus 20 includes a tempering furnace 1 and a tempering cooling unit 2 aligned consecutively in the direction of travel of the glass sheet in accordance with figure 3. The tempering furnace 1 shown in figure 3 is equipped with horizontal rotating rollers. The rotating rollers form the conveyor line, the so-called roller line. Alternatively, an air jet levitation table (not shown in figure 3) can be used. The tempering apparatus is further equipped with a temperature scanner 5 and a pyrometer 8 fitted between the tempering furnace and the tempering cooling unit above or below the conveyor line. The temperature scanner 5 has been fitted to the tempering apparatus in such a way that the temperature scanner can sight the glass sheet moving on the conveyor line through its lens without a mirror. A glass sheet G to be heated is moved in the tempering furnace at a predetermined speed in the same direction, or back and forth, according to the tempering instructions, tempering recipe or the like predetermined instructions chosen based on the properties of the glass sheet, wherein, for example, the temperature of the tempering furnace and the heating time of the glass sheet to be tempered in the tempering furnace are defined. The conveying speed of the tempering furnace on the conveyor line is adjustable. The heated glass sheet moves from the tempering furnace 1 to a tempering cooling unit 2 at a conveying speed W, which is typically higher than the conveying speed of the glass sheet inside the tempering furnace 1. Typically, the conveying speed W from the tempering furnace to the tempering cooling unit is in the range between 200 and 800 mm/s.

The tempering cooling unit 2 shown in figure 3 is equipped with horizontal rotating rollers 3. The rotating rollers form the glass sheet conveyor line that can be a similar conveyor line to the one in the tempering furnace. The conveyor has been provided through the tempering cooling unit. The tempering cooling unit 2 has further been equipped with cooling air housings 4 provided above and below the conveyor line. The cooling air housings 4 have been equipped with blow holes 7, from which the cooling air is released as air jets towards the glass sheet G. The blow holes 7 are typically round-shaped holes. In the cooling air housings 4, the blow holes 7 have typically been positioned in consecutive rows in the direction of the cooling air housing. The blow holes 7 can also be of another shape, for example slits. The cooling capacity of the cooling air jets being released from the cooling air housings is adjusted by altering the blowing pressure Δp and/or blowing distance H. Blowing pressure, i.e. the pressure difference between the internal pressure of the cooling air housing and the ambient pressure, is measured with a pressure sensor 6 inside the cooling air housing 4.

When the tempering furnace 1 is an air jet levitation furnace, the rollers 3 or the air jet levitation table along with its conveyor members are in the tempering cooling unit 2 typically in a slightly tilted position with regard to the horizontal direction perpendicular to the direction of movement of the glass sheet G.

During tempering, each part of a glass with a thickness of 3 mm, for example, must be in the tempering cooling for at least about 3 seconds. For example, at a tempering cooling conveying speed of 600 mm/s, this would require a pass-through type tempering cooling unit of at least 1800 mm in length. In a pass-through type tempering cooling unit, a glass sheet moves in only one direction at a conveying speed W through a tempering cooling unit. A so-called oscillating tempering cooling unit is usually about 1 m longer than the longest allowed glass loading length. Thus, the glass loading moves at a conveying speed W in its entirety into the tempering cooling unit. The glass loading turns back around as the front of the glass loading reaches the other end of the tempering cooling unit. After this, the glass loading moves back and forth in the tempering cooling unit 2, until the tempering cooling and usually also the final cooling has been completed. In tempering cooling, the glass sheet is cooled from the tempering temperature to a temperature of about 450 °C, and in the final cooling, from this to a temperature of about 50 °C. The final cooling does not affect the residual stresses of the glass.

Figure 4 shows a tempering apparatus according to another embodiment. In this embodiment, a temperature scanner 5 has been fitted in the bottom part of the tempering cooling unit in such a way that the temperature scanner can sight the glass sheet G moving on the conveyor line through a mirror 9 installed at an angle in front of the temperature scanner lens at a distance from the lens. The problem with the bottom temperature scanner shown in figures 3 and 4 is the contamination of its optical surfaces. A dusty lens or mirror may interfere with the measuring and/or cause inaccuracies in the readings. However, to prevent contamination of the optical surfaces, the temperature scanner 5 can be equipped with an automatic lens or mirror cleaning apparatus that blows and/or wipes their surfaces clean of dirt and dust. Data from the pyrometer 8 measuring the temperature of the top surface of the glass sheet is used to continuously calibrate the temperature scanner 5. It gives accurate information on the actual temperature of the glass sheet each time an uncoated glass G enters its measuring radius. In the above-mentioned calibration, this reading is compared to the reading from the bottom temperature scanner 5 from the corresponding measurement site on the glass sheet, and a setting of the bottom temperature scanner, such as the reflection coefficient of the mirror 9, is changed based on the comparison.

Figure 5 shows a block diagram of a tempering apparatus. In figure 5, the parts of the assembly of the tempering apparatus line and the primary data streams in the method are shown. Figure 5 shows a tempering furnace 1, and in front of it, a loading table 13, onto which the glass loading to be tempered, i.e. a glass sheet or a plurality of glass sheets, is positioned, a tempering cooling unit 2, and behind it, an unloading table 14, from which the tempered glass sheets are lifted onto glass trestles. The tempering line control unit 11 is typically located adjacent to the beginning of the loading table. In figure 5, an alarm device 12 is located adjacent to the end of the unloading table 14. The alarm device 12 can be a warning light, a buzzer or a display terminal, for example. The location of the calculating unit 10 has been shown in figure 5 for the sake of clarity in depicting the data streams. Typically, the calculating unit 10 is located in the same space as the control unit 11 or even on a so-called cloud server provided outside the glass plant.

In the calculating unit 10, a tempering level is calculated for at least one, preferably for each glass sheet of a glass loading. The tempering level includes the compressive stress at the glass sheet surface, the highest tensile stress of the interior, or the entire thickness direction residual stress profile. The thermal and mechanical properties of the glass sheet are the initial data for the calculation programmed into the calculating program used by the calculating unit. Thus, these properties are already known before the glass sheet is tempered. The primary properties specific to a glass or a glass loading used in the calculation, the initial data for the calculation, are the glass sheet thickness L, time in the tempering cooling unit t_{tc}, the tempering temperature field T_{i,y-z} of the glass sheets and the heat transfer coefficient h achieved with the cooling air jets at the surface of the glass sheet. The heat transfer coefficient h is dependent particularly on the blowing pressure Δp of the air jets. The glass sheet thickness L is typically in the range between 3 and 19 mm and almost always in the range between 2 and 25 mm.

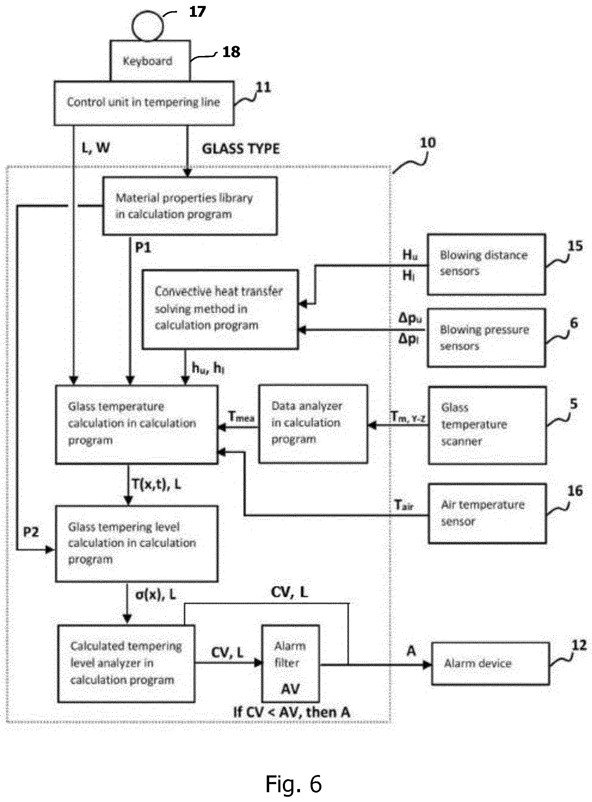

Figure 6 shows a flow chart according to an embodiment of the present invention. In the first step in figure 6, the properties of the glass sheet to be tempered are identified, the most important of which is the glass sheet thickness L. The glass sheet thickness L is entered into the control unit 11 by a user, for example by an operator 17, and based on the glass sheet properties identified, the tempering instructions are determined, i.e. a heating recipe, instructions on the values, such as the temperature of the tempering furnace and the glass sheet heating time, with which the glass sheet is to be tempered in the tempering furnace, and a cooling recipe, instructions on the values, for example the blowing pressure and the blowing distance in the tempering cooling unit, with which the glass sheet is to be cooled, and on the conveying speed S at which the glass sheet is to move from the tempering furnace to the tempering cooling unit and/or how long the tempering cooling time t_{tc} is. Entering the data can be done using, for example, a keyboard 18 or a wireless application, from which the data is transferred to the tempering apparatus. Alternatively, information on the glass sheet properties can be identified automatically, for example the information concerning the thickness can be read from an automatic glass sheet thickness measuring device, from which the information read is transferred to the control unit 11. When choosing the glass sheet tempering instructions, i.e. the heating and tempering cooling recipes, the operator also enters information on the type of glass sheet into the control unit. The glass sheet can be of regular clear glass type or of another type of glass: uncoated glass, coated selective glass or glass coated with another type of coating. The type of the glass sheet is also a property that affects particularly the heating, but usually also the cooling recipe. Further, based on the glass sheet type, the calculating unit 10 will select from its material properties library the correct glass sheet thermal properties, in step P1, and the mechanical material properties in step P2. Information on the residence time t_{tc} of the glass sheet, particularly of each part of the glass sheet, in the tempering cooling unit can be transferred to the calculating unit 10 from the control system 11. In its simplest form, t_{tc} = S / W, wherein S is the length of the tempering cooling unit (constant) and W is the conveying speed. In the above-mentioned oscillating tempering cooling unit, calculating the residence time t_{tc} is only slightly more complicated. The residence time can also be measured by means of photocells, for example.

Above, a few control variables relating to the furnace heating recipe were listed. The method determines the tempering level of the glass sheet on the basis of the measured tempering furnace heating result, i.e. the temperature of the glass sheet. The tempering temperature is measured after the heating, before the glass sheet enters the tempering cooling. If the heating of the subsequent glass loading is to be increased, for example based on the calculated tempering level, then, for example, the heating time of the furnace is increased. Increasing the temperature level of the tempering furnace is a slightly slower control means than increasing the heating time. The temperature of the tempering furnace can also be adjusted locally, as the temperature furnace is equipped with a separately adjustable matrix-like heating resistor field. In tempering furnaces equipped with convection-based heating elements, heating can also be adjusted by changing the blowing pressure or time of the hot air jets. The size of the glass sheet (length and width) is a property that usually affects the heating recipe. The size of the glass sheet usually hardly affects the cooling recipe.

The heat transfer coefficient h, achieved with the cooling air jets at the surface of the glass sheet, is an essential piece of information in calculating the tempering level. The best way to determine it is by measurements. The heat transfer coefficient can be measured, for example, by cooling a heated thick copper plate in a tempering cooling unit. The temperature sensor inside the copper will thus produce a cooling curve from which the heat transfer coefficient can be determined to a relative degree of accuracy. More information on this type of measurement is provided in the publication Rantala, M 2015, Heat Transfer Phenomena in Float Glass Heat Treatment Processes. Tampere University of Technology. Publication, Volume 1355, Tampere University of Technology. Series of measurements where copper is cooled at various blowing pressures and blowing distances provide sufficient information for tabulating the heat transfer coefficients with relation to the blowing pressure and blowing distance. The calculating unit will thus determine the heat transfer coefficient h from the table (directly or by interpolating from the nearest tabulated values) for the top and bottom surface of the glass (values can be different, upper surface hᵤ and lower surface hi) based on the blowing pressure Δp measured with a pressure sensor 6 (in the nozzle housings above and below the glass) and the blowing distance H set (chosen by operator using the control unit 11). The tempering cooling unit may also contain measuring devices for measuring the blowing distance H, whereby the calculating unit 10 receives the blowing distance H from the blowing distance measuring device 15. Generally, the blowing distance H set corresponds closely to the actual blowing distance when devices controlling the blowing distance are calibrated at deployment. Typically, the blowing pressure is from 10 to 20 000 Pa depending on the glass thickness. The above-mentioned lower end of the pressure range is typical for a glass sheet with a thickness of 19 mm and the pressure of the upper limit for a glass sheet with a thickness of 3 mm. Typically, the blowing distance, i.e. the shortest distance between the blow hole and the glass sheet, is from 10 to 40 mm depending on the glass sheet thickness. The heat transfer coefficient h can also be determined based on measurement-based correlation equations found in literature. The variables in the equations are the blowing pressure and the blowing distance. Furthermore, information on the system dimensions (diameter of the blow hole, distance between the blow holes, inter alia) is required. However, the correlation equations may have been defined using a system that is too different from the tempering cooling unit of the present invention. Therefore, the accuracy of the correlation equations may not be sufficient. Instead of the above-mentioned measurements, numerical modelling of flow dynamics (CFD) may be used to tabulate the heat transfer coefficients (with relation to Δp and H), as long as the accuracy of the modelling is confirmed with at least a few of the above-mentioned measurements. Typically, a heat transfer coefficient of h = 40 W/(m²K) is sufficient for tempering a glass sheet with a thickness of 19 mm and a heat transfer coefficient h = 650 W/(m²K) is sufficient for tempering a glass sheet with a thickness of 3 mm, but this sufficiency is also dependent on the tempering temperature of the glass sheet. However, typically, the heat transfer coefficient is in the range between 40 and 650 W/(m²K). Typically, when the thickness of the glass to be tempered changes, the blowing pressure Δp and the blowing distance H are adjusted so as to change the heat transfer coefficient h. Often changing only the blowing pressure, and in some cases, only the blowing distance, is sufficient. The blowing pressure is increased when the heat transfer coefficient is to be increased. The blowing distance is shortened when the heat transfer coefficient is to be increased.

Careful deployment of the blower and determining of the blower curves during the deployment of the tempering apparatus reduce the need for a measured blowing pressure Δp in calculating the tempering level. Based on the blower curves, the blowing pressure can be determined based on the blower impeller rotation speed, and the blowing pressure set by the operator corresponds closely to the actual blowing pressure. Thus, the calculating unit 10 can use the value set by the operator in the control unit 11 as the blowing pressure Δp. In a preferred solution, the calculating unit 10 receives the blowing pressure information from the pressure sensor 6.

In a preferred solution according to figure 6, the blowing pressure is measured from both sides of the glass sheet G, as the blowing pressure of the top and bottom blow may vary significantly when the blowing air originates from different blowers, for example. In this case, the pressure measuring devices measure the blowing pressure at the top and at the bottom (Δpᵤ, Δpₗ). Further, the blowing distances may vary significantly, hence, in a preferred solution, they are also measured from both sides of the glass sheet. Thus, the distance measuring devices measure the blowing distance at the top and at the bottom (Hᵤ, Hₗ).

In figure 6, the method of determining the heat transfer coefficient in the calculating unit (above, different alternatives to the method were described) determines the heat transfer coefficient at the top hᵤ based on the blowing pressure Δpᵤ and the blowing distance Hᵤ, and the heat transfer coefficient at the bottom hₗ based on the blowing pressure Δpₗ and the blowing distance Hi.

The calculating unit will also require information on the tempering air temperature Tₐᵢᵣ. A typical plant floor temperature of 25 °C can be used, particularly if the suction inlets of the tempering blower are inside the plant. The tempering air temperature may also vary significantly depending on the season and the weather, whereby using a measured temperature improves the accuracy of the calculation. In a preferred solution, this information is received by the control unit 10 from a temperature sensor 16 at the suction inlet or in the blower duct, in figure 6.

In the method for calculating the tempering level, a temperature field T_{i,y-z}, measured from the glass sheet with a temperature scanner after the furnace and before the start of the tempering cooling, is used, which information is passed from the temperature scanner 5 to the calculating unit 10. In a preferred solution, the temperature scanner measures the temperature from the bottom surface of the glass sheet. It is also preferred that the temperature at the top surface of the glass sheet is measured with a pyrometer, the reading from which is used to continuously calibrate the bottom temperature scanner. A temperature scanner at the top can also be used to continuously calibrate the temperature scanner, but the costs associated with a pyrometer are lower.

Figures 2a and 2b illustrate the method of measuring the temperature in a glass sheet according to the present invention. The temperature field measured by with temperature scanner as shown in figure 2a consists of small pixels, the size of which is determined by the properties of the scanner. Each pixel has its own measured temperature T_{i,y-z}. In the simplest solution, the average temperature of the pixels in the entire glass loading, i.e. the average temperature of the entire glass loading T_{m, loading}, is selected, for example by the calculating program of the calculating unit, as the measured tempering temperature Tₘₑₐ of the glass sheet. In a preferred solution, in figure 2a, the average temperature of all pixels within a glass sheet, i.e. the average temperature Tₘ of the entire glass sheet, is selected, for example by the calculating program of the calculating unit, as the measured tempering temperature Tₘₑₐ of the glass sheet. The glass sheet can also be divided into smaller subsections, as presented in figure 2b, whereby the reference temperature of the subsection T_{m, Y-Z} is the average temperature of the pixels therein. The size of the subsection is typically approximately in the range between 4 x 4 cm and 30 x 30 cm. The size of the subsection may be dependent on the size of the glass sheet, and it does not have to be square. In counting the average, values appearing to be incorrect can be filtered out from the pixel temperatures. The lowest value T_{m, Y-Z}, min among the reference temperatures T_{m, Y-Z} is selected as the measured tempering temperature Tₘₑₐ of the glass sheet. A measured tempering temperature Tₘₑₐ (= T_{m, loading} or Tₘ or Tₘ, _{Y-Z, min}) representing the glass sheet is selected for each of the glass sheets in the glass loadings in the above-described manner. The tempering temperature can also be selected based on the temperature scanner measurement data in some other manner. It is essential that the temperature selection is based on the temperature scanner measurement data generally denoted by Tₘₑₐ. Thus, the temperature data analysis program of the calculating unit determines from the temperature field T_{i,y-z} measured with a temperature scanner the measured tempering temperature Tₘₑₐ representing the glass sheet, used by the tempering level calculating program of the calculating unit as the tempering temperature T₀ of the glass sheet.

The temperature scanner can also be located above the glass, in which case it measures the surface temperature field of the top surface of the glasses. However, measurement from the top cannot be carried out for glasses with a coating on the top surface where the top surface emissivity is low (selective, i.e. low-e glass) and unknown. In a preferred solution, the temperature is measured with a temperature scanner at the bottom.

In some continuous tempering apparatuses, the glass sheets move sequentially and in approximately the same position across the width of the line. In this case, it is possible to measure the glass temperature rather comprehensively using one or at least a few pyrometers. One pyrometer measures the glass temperature in one line in the direction of the glass movement. In the simplest solution, the temperature scanner is a pyrometer or another measuring device measuring the glass temperature in a comparable manner. In this case, the measured tempering temperature Tₘₑₐ representing the glass sheet is the average temperature of the glass in one or a plurality of lines in the direction the glass movement. In a preferred solution, the temperature is measured with a temperature scanner measuring the temperature of the entire surface area of each glass in a glass loading.

A program calculating the glass temperature in the calculating unit determines the development of the thickness direction temperature profile T(x,t) of the glass during the tempering cooling from the energy equation (1): ${ρc}_{p} \frac{\partial T}{\partial t} = \frac{\partial }{\partial x} \left(k\frac{\partial T}{\partial x}\right) + \frac{\partial {q}_{r}}{\partial x}$ (1), the boundary conditions of which are the equations (2) and (3): $k \frac{\partial T}{\partial x} \left(0, t\right) = {q}_{r , u} + {q}_{c , u}$ (2) $- k \frac{\partial T}{\partial x} \left(L, t\right) = {q}_{r , l} + {q}_{c , l} + {q}_{ct}$ (3), where T = temperature, L = thickness of the glass sheet, k = thermal conductivity of the glass, ρ = density of the glass and cₚ = specific heat capacity of the glass.

Furthermore, the initial temperature of the glass sheet (or a subsection of the glass sheet) T(x, 0) = T₀. The tempering temperature T₀ of the glass sheet is the tempering temperature Tₘₑₐ measured from the surface of the glass sheet with a temperature scanner and determined from the measured temperature field of the glass sheet, for example in the above-described manner. The tempering temperature may account for natural cooling ΔTₘ of the glass sheet between the temperature scanner 5 and the tempering cooling unit. The residence time from the temperature scanner to the tempering cooling unit is typically from 0,1 to 0,7 s depending on the conveying speed of the conveyor line. Thus, T₀ = Tₘₑₐ - ΔTₘ, wherein Tₘₑₐ is the glass sheet temperature based on the temperature scanner measurement. ΔTₘ is in the range between 0 and 10 °C. The calculation is carried out over glass sheet thickness L. At the top surface of the glass, the thickness direction coordinate x = 0 and at the bottom surface x = L.

In the study of heat transfer, solving the energy equation is a common problem for which several solutions have been presented in literature, in addition to the above-mentioned reference, Rantala 2015, for example in the following publications: Field, R. E. and Viskanta, R., Measurement and prediction of the dynamic temperature distributions in soda-lime glass plates, Journal of American Ceramic Society, vol. 73, 7, pp. 2047-2053, 1990; Gardon, R., Calculation of temperature distribution in glass plates undergoing heat treatment, Journal of American Ceramic Society, vol. 41, 6, pp. 200-209, 1958; Siedow, N., Lochegnies, D., Gro-san, T. and Romero, E., Application of a New Method for Radiative Heat Transfer to Flat Glass Tempering, Journal of American Ceramic Society, vol. 88, 8, pp. 2181-2187, 2005.

The thermal material properties of the glass required in the equation are density ρ, specific heat capacity cₚ and thermal conductivity k. They are commonly known and have been provided for the calculating unit in the calculating program. These and the radiation characteristics of the glass have been denoted by P1 in figure 6.

In the equations, q_{r,u} is the net radiation heat flow upwards (downwards q_{r,l}) between the glass and the environment. The method for determining it has been disclosed and the glass radiation characteristics required for it have been disclosed in the above-mentioned publications.

The terms q_{c,u} and q_{c,l} are the convective cooling powers achieved at the top and bottom surface of the glass with the air jets of the tempering cooling unit, calculated from the equations q_{c,u} = hᵤ[Tₐᵢᵣ-T(0,t)] and q_{c,l} = hₗ[Tₐᵢᵣ-T(L,t)]. In the equations, hᵤ and hₗ are the convective heat transfer coefficients at the top and bottom surface of the glass. Three alternative techniques for determining them were presented above. It is essential that the heat transfer coefficients have been tabulated or presented by way of equations with relation to the blowing pressure and blowing distance. Thus, the calculating unit is able to determine them to a sufficient degree of accuracy based on the measured blowing pressure and distance. In the equations, Tₐᵢᵣ is the temperature of the tempering air. Included in the boundary condition for the bottom surface is also the term q_{ct} that takes into account the heat conducted from the glass (at contact points between the glass and the roller) transferring to the roller. Typically, string with poor thermal conductivity has been coiled around the rollers of the tempering cooling unit. In this case, the effect of the contact heat transfer can be rounded to the value q_{ct} = 0.

With the energy equation (1), the development of the thickness direction temperature profile T(x,t) of the glass sheet during the time interval 0 ≤ t ≤ t_{tc}, where t_{tc} is the residence time of the glass sheet (point in glass) in the tempering cooling, is calculated. Typically, it is obtained in the above-described manner based on the glass sheet conveying speed and the length of the tempering cooling unit. The calculating period t_{tc} must, however, be long enough for the temperature of the entire thickness of the glass sheet to fall below the temperature limit T_{tcend}. Cooling beyond this temperature no longer affects the residual stress profile formed in the glass sheet, i.e. the tempering level. Time t_{tc} is thus the time at which the glass sheet has cooled to below the temperature limit T_{tcend}. A typical temperature limit T_{tcend} = 450 °C. If the value of the convective heat transfer coefficient (hᵤ, hₗ) changes within the time interval 0 ≤ t ≤ t_{tc}, it will be accounted for in the calculation.

As a solution to the energy equation (1), a thickness direction temperature profile T(x,t) of the glass sheet or a subsection of the glass sheet over the tempering cooling time (0 - t_{tc}) at time intervals Δt, is obtained. Time interval Δt is in the range between 0.001 and 2 s depending on the glass thickness. The temperature profile is obtained at the density of thickness interval Δx over glass thickness L. The thickness interval Δx is (0.01 - 0.2)L. Then, the temperature profile T(x,t) calculated from the energy equation (1) is used to calculate the residual stresses of the glass sheet. The tempering level achieved in the glass sheet during tempering is calculated from the equation: $\begin{matrix}{σ}_{ij} \left(x, t\right) = {δ}_{ij} {\int }_{0}^{t} K \left(t-t′\right) \frac{\partial \left(\overline{ε}\left(x, t\right)-3{ε}^{th}\left(x, t\right)\right)}{\partial t ′} dt ′ \\ + 2 {\int }_{0}^{t} G \left(t-t′\right) \frac{\partial \left({ε}_{ij}\left(x, t\right)\right)}{\partial t ′} dt ′\end{matrix}$ where the initial condition is σᵢⱼ (x,0) = 0 and the load due to by thermal elongation ε^{th}(x,t) caused by cooling is calculated by means of the temperature profile T(x,t) calculated from the energy equation (1): ${ε}^{th} \left(x, t\right) = \left({α}_{l}-{α}_{g}\right) \left({T}_{f}\left(x, t\right)-{T}_{f}\left(x, 0\right)\right) + {α}_{g} \left(T\left(x, t\right)-T\left(x, 0\right)\right)$

In the thermal elongation equation, the fictitious temperature T_{f} is calculated by means of the temperature profile T(x,t) and material parameters Mₚ: ${T}_{f} \left(t\right) = T \left(t\right) - {\int }_{0}^{t} {M}_{p} \left(t-t′\right) \frac{\partial T \left(t\right)}{\partial t ′} dt ′$

The stress field must satisfy the general equilibrium conditions: ${\int }_{0}^{L} {σ}_{yy} \left(x, t\right) dx = {\int }_{0}^{L} {σ}_{zz} \left(x, t\right) dx = 0$ and ${\int }_{0}^{L} {σ}_{yy} \left(x, t\right) \left(x-L/2\right) dx = {\int }_{0}^{L} {σ}_{zz} \left(x, t\right) \left(x-L/2\right) dx = 0$

Solving the tempering stresses of glass, i.e. the above equations, is a common problem in the study of mechanics of glass, discussed for example in the publications Aronen, A 2012, Modelling of Deformations and Stresses in Glass Tempering. Tampere University of Technology. Publication, Volume. 1036, Tampere University of Technology; Nielsen, J.H., 2009, Tempered Glass - Bolted Connections and Related Problems, Ph.D. thesis, DTU Civil Engineering, Lyngby, Denmark; Carrè, H., 1996, Numerical Simulation of Soda-Lime Silicate Glass Tempering, Journal de Physique IV, vol. 6, no. 1, pp. 175-185.

The elongations presented in the stress equation are hydrostatic elongation ε̅ and deviatoric elongation εᵢⱼ. The mechanical material properties of the glass required in the equations are the time- and temperature-dependent compression modulus K and the shear modulus G and the heat expansion coefficients αₗ and α_{g}. They are commonly known for example from the above-mentioned reference, Aronen 2012, and they have been provided for the calculating unit in the calculating program. The mechanical properties of the glass sheet have been denoted by P2 in figure 6.

As a result of the calculation, the thickness direction residual stress profile σ(x) of the glass sheet, i.e. the stress σ value over glass thickness at thickness intervals ΔL, is obtained. The residual stress profile determines the tempering level of the glass. In tempering a glass sheet, the improved strength is represented particularly by residual stress at the surface of the glass, i.e. the smaller of the values σ(0) or σ(L). The safety of the tempered glass sheet when the glass sheet breaks, for example as the particle count calculated according to the standard EN12150-1, is dependent particularly on the amount of tensile stress σ(0,5L) at the center thickness of the glass x=0,5L.

Based on the calculated residual stress profile, a reference variable for the tempering level of the glass is selected. The reference variable is based on the calculated thickness direction residual stress profile σ(x) of the glass. In a preferred case, the reference variable is the residual stress at the center thickness of the glass, i.e. σ(0,5L). The reference variable may also be the residual stress at the surface of the glass, i.e. σ(0) or σ(L), or a type of average value or some other residual stress value calculated from the residual stress profile σ(x). The general notation for the reference variable of residual stress is σ_{cal}. Residual stress at the center thickness of the glass, i.e. σ(0,5L), can also be converted to the glass particle count according to the standard EN12150-1 and used as a reference variable. The change can be made, for example, based on the result images published in the above-mentioned reference, Akeyoshi 1965, wherein the particle count has been presented with relation to stress σ(0,5L) for several different glass thicknesses. The information from the result images can be provided for the calculating unit, for example, by using polynomial fitting. The applicant's knowledge on the relationship between stress σ(0,5L) and the particle count is based on, in addition to literature, extensive research findings gathered by the applicant. In the research, a large number of glasses with various thicknesses were tempered, their σ(0,5L) value measured using the above-mentioned SCALP measuring device and the particle count assay according to the standard EN12150-1 performed on the glasses. The general notation for the reference variable is CV, covering the value σ_{cal} and the particle count N_{cal} determined on the basis thereof.

The value of the reference variable CV is used to ensure the successful tempering of the glasses of a glass loading to safety glass. The monitoring is carried out by an operator based on the reference variable CV displayed for the glass on the display of an alarm device 12 or an alarm given by the alarm device 12. The difference between the options is that in the former, the operator is capable of concluding whether the tempered glass is safety glass based on the reference variable CV, for example σ(0,5L). In the latter, the conclusion is reached by the calculating unit, whereby the glass sheet is found not to qualify as safety glass based on an alarm given by the alarm device. The former is possible when the value of the reference variable CV is displayed on the alarm device display. More on the latter in the following.

Based on the calculated residual stress profile, a reference variable CV for the glass sheet tempering level is selected and then compared to a predetermined alarm limit value AV. On the condition that the comparison between the reference variable and the alarm limit value fulfils a predetermined criterion, the calculating unit creates an alarm and sends an alarm message A to the alarm device. The predetermined criterion may be a deviation between the reference variable and the alarm limit value. For example, a deviation of the reference variable from a standard alarm limit value or a deviation of the reference variable from another predetermined alarm limit value. An alarm can be created, for example, if the value of the calculated reference variable CV is smaller than the value of the alarm limit AV. In the context of the present invention, the alarm limit value can also be referred to as a threshold value.

The alarm limits AV have been programmed into the calculating unit. The alarm limit value is, for example, 45 MPa, if the reference variable is stress σ(0,5L). The alarm limit value is, for example, 60 particles, if the reference variable is the particle count according to EN12150-1, at least 40 particles. By comparing the reference variable to the alarm limit value, information on whether the glass sheet fulfils the requirements of safety glass standards is obtained. Preferably, the alarm limit of the particle count is at least 50 % higher than the minimum (e.g. 40) presented in the standard EN12150-1 (and table 1), i.e. for example 60, when the glass thickness is 4 to 12 mm. Typically, the alarm limit value is in the range between 50 to 80 particles, when the glass thickness is 3.8 mm or more. Typically, the alarm limit value is in the range between 25 to 50 particles, when the glass thickness is under 3.8 mm. Preferably, the stress σ(0,5L) alarm limit value is high enough for, on the basis thereof, at least 50 % more particles to be formed in the glass compared to what the minimum requirement for passing the standard EN12150-1 requires. Typically, the alarm limit σ(0,5L) value is in the range between 45 and 60 MPa (tensile stress), when the glass thickness is 3 to 4 mm. Typically, the alarm limit σ(0,5L) value is in the range between 35 and 55 MPa, when the glass thickness is 5 to 8 mm. Typically, the alarm limit σ(0,5L) value is in the range between 30 and 45 MPa, when the glass thickness is 10 - 19 mm. When the reference variable is the residual stress at the glass surface, i.e. σ(0) or σ(L), the alarm limit is typically 70 to 110 MPa (compressive stress), or 1.8 times the absolute value of the above-mentioned alarm limit σ(0,5L).

When the alarm device raises an alarm with light, sound, color or a message displayed on the display screen, the reference variable for the glass loading or for at least one of the glass sheets in the glass loading is below the alarm limit. It is preferred that the display screen identifies these glass sheets so that finding them among the glass sheets of the glass loading is easy. Based on this, the tempering line operator can remove the glass sheets raising the alarm from among the production glasses. The standard safety testing of these glass sheets gives information with which the above-described method for ensuring the safety of tempered glass sheets can be refined. Based on this information, the difference between the alarm limit and the standard minimum can be reduced.

By using the calculating program, it can be indicated on the display terminal, for example by way of colors, if the glass loading or any of the glass sheets in the glass loading does not qualify as safety glass or if it is safety glass. An alarm indicating the presence of safety glass that does not qualify as safety glass can be given, for example, with a color on the glass loading pattern displayed on the display terminal, for example red. The calculating program can also indicate, e.g. on the display terminal, the level of the reference variable using preselected colors. For example, the color green can indicate that the glass sheet or the glass loading is safety glass. For example, the color yellow can indicate that the glass sheet or glass is likely to be safety glass. For example, the color red can indicate that the glass sheet or the glass loading is not safety glass. The above-described use of three colors requires setting two limit value threshold values AV, i.e. threshold values AV1 and AV2. Thus, for example glass sheets with the reference variable above the higher threshold value AV2 are considered safety glass, and glass sheets with the reference variable CV below the lower threshold value AV1 are considered not to qualify as safety glass. Glass sheets with the reference variable CV in the range between AV1 and AV2 are considered likely to be safety glass. The calculating program can also indicate the level of the reference variable with colors. This can be carried out, for example, by positioning a reference variable CV chosen for each glass on top of the glass sheet temperature scanner image or another pattern indicating the border of the glass sheet and highlighting it with a predetermined color. The predetermined colors can be for example red, yellow and green. For example, if the reference variable value would be in the range between 49 and 50 MPa, and the reference variable in this range would be acceptable as safety glass, this could be presented in the temperature scanner images with a color chosen by the user, for example green.

The method can be used to ensure the successful tempering of a glass loading to safety glass according to various glass sheet thicknesses and glass sheet types. Thus, for example, as the calculated tempering level (CV) approaches the alarm limit (AV), when the predetermined criterion is fulfilled, the selected temperature and/or heating time of the tempering furnace, i.e., the conveyor line speed, is readjusted. Respectively, as the calculated tempering level approaches the alarm limit, when the predetermined criterion is fulfilled, the selected blowing pressure and/or blowing distance of the air jets in the tempering cooling unit can be readjusted when the predetermined criterion is fulfilled. The tempering furnace control system of the tempering apparatus can automatically increase/decrease the glass sheet heating time in the furnace, or suggest it to be increased/decreased, if a deviation between the tempering level and the alarm limit is detected. Further, the tempering cooling unit control system of the tempering apparatus can automatically suggest the blowing pressure and/or the blowing distance of the glass sheet cooling jets to be adjusted if a deviation between the tempering level and the alarm limit is detected.

## Claims

1. A method for tempering a glass sheet with a tempering apparatus (20) and for monitoring the tempering level of a tempered glass sheet during production, which method comprises:
- identifying properties of the glass sheet, said properties preferably including the glass sheet thickness (L),
- based on the properties identified, adjusting the temperature of the tempering furnace (1) and/or the heating time of the glass sheet (G), adjusting the blowing pressure and/or blowing distance of the air jets of the tempering cooling unit (2),
- heating the glass sheet in a tempering furnace,
- measuring the achieved temperature of the glass sheet at the surface of the glass sheet after the tempering furnace, and before the cooling unit
- cooling the glass sheet in a tempering cooling unit,
**characterized in that** in the method, in the calculating unit (10) connected to the tempering apparatus (20):
- calculating, based on the measured temperature of the glass sheet, the development of the thickness direction temperature profile of the glass sheet during the tempering cooling, and calculating, based on the development of the thickness direction temperature profile calculated, the residual thickness-wise stress profile achieved in the glass sheet during the tempering,
- selecting, based on the calculated thickness-wise residual stress profile, a glass sheet tempering level reference variable, where the glass sheet tempering level reference variable is preferably the residual stress at the center thickness of the glass, σ(0.5L), or the residual stress at the surface of the glass, σ(0) or σ(L), or a type of average value or some other residual stress value calculated from the residual stress profile σ(x), presenting the tempering level reference variable on a display terminal (12), and/or comparing the selected reference variable to a predetermined threshold value, and on the condition that the comparison between the reference variable and the threshold value fulfils a predetermined criterion, creating an alarm and/or detecting the glass sheet not to qualify as safety glass, presenting the alarm and/or information detecting that the glass sheet does not qualify as safety glass on a display terminal (12).

2. A method according to claim 1, **characterized in that** the predetermined criterion is a deviation between the reference variable and the threshold value.

3. A method according to claim 1, **characterized in that** the temperature of the tempering furnace (1) and/or the heating time are readjusted when the predetermined criterion is fulfilled.

4. A method according to claim 1, **characterized in that** the blowing pressure and/or the blowing distance of the air jets of the tempering cooling unit (2) are readjusted when the predetermined criterion is fulfilled.

5. A method according to claim 1, **characterized in that** the reference variable is proportional to the particle count of the glass sheet.

6. A method according to claim 1, **characterized in that** the tempering level reference variable is the calculated residual stress profile at the center thickness of the glass sheet, σ(0,5L), or a particle count N_{cal} of the glass sheet calculated on the basis thereof.

7. A method according to claim 1, **characterized in that** the achieved temperature of the glass loading is measured from the entire surface area of the glass loading, and from the measured temperature field (T_{i,y-z}), the average of all measurement points within the temperature field is selected as the measured tempering temperature (Tₘₑₐ) of the glass sheets of a glass loading.

8. A method according to claim 1, **characterized in that** the achieved temperature of the glass sheet is measured from the entire surface area of the glass sheet, and from the measured temperature field (T_{i,y-z}), the average of measurement points within the temperature field or a subsection thereof is selected as the measured tempering temperature (Tₘₑₐ) of the glass sheet.

9. A method according to claim 1, **characterized in that** the surfaces of the glass sheet are cooled with air jets, the convective cooling powers (q_{c,u} and q_{c,l}) of which are calculated from the equation q_{c,u} = hᵤ[Tₐᵢᵣ-T(0,t)] and q_{c,l} = hₗ[Tₐᵢᵣ-T(L,t)].

10. A method according to claim 9, **characterized in that** the measured blowing pressures (pᵤ, pi) are used as initial data for calculating the convective heat transfer coefficient (hᵤ, hₗ).

11. A method according to claim 9, **characterized in that** the measured blowing distances (Hᵤ, Hₗ) are used as initial data for calculating the convective heat transfer coefficient (hᵤ, hₗ).

12. A method according to claim 9, **characterized in that** the measured air jet temperature Tₐᵢᵣ is used for calculating the convective cooling power of the glass sheet surface.

13. A method according to claim 1, **characterized in that** the achieved temperature of the glass sheet is measured from the bottom surface of the glass sheet.

## Patentansprüche

1. Verfahren zum Härten einer Glasscheibe mit einer Härtungsvorrichtung (20) und zum Überwachen des Härtungsgrades einer gehärteten Glasscheibe während der Herstellung, wobei das Verfahren umfasst:
- Identifizieren von Eigenschaften der Glasscheibe, wobei die Eigenschaften vorzugsweise die Glasscheibendicke (L) umfassen,
- basierend auf den identifizierten Eigenschaften Anpassen der Temperatur des Härteofens (1) und/oder der Erwärmungszeit der Glasscheibe (G), Anpassen des Blasdruck und/oder des Blasabstands der Luftdüsen der Härtungskühleinheit (2),
- Erwärmen der Glasscheibe in einem Härteofen,
- Messen der erreichten Temperatur der Glasscheibe auf der Oberfläche der Glasscheibe nach dem Härteofen und vor der Kühleinheit,
- Abkühlen der Glasscheibe in einer Härtungskühleinheit,
**gekennzeichnet durch**, im Verfahren in der mit der Härtevorrichtung (20) verbundenen Recheneinheit (10):
- Berechnen der Entwicklung des Dickenrichtungstemperatur-Profils der Glasscheibe während der Härtungsabkühlung basierend auf der gemessenen Temperatur der Glasscheibe und Berechnen des in der Glasscheibe während des Härtens erzielten Restdickenspannungs-Profils basierend auf der berechneten Entwicklung des Dickenrichtungstemperatur-Profils,
- Auswählen einer Glasscheiben-Härtungsgrad-Referenzvariable basierend auf dem berechneten Dickenrestspannungs-Profil, wobei die Glasscheiben-Härtungsgrad-Referenzvariable vorzugsweise die Restspannung an der Mittendicke des Glases, σ(0.5L), oder die Restspannung an der Oberfläche des Glases, σ(0) oder σ(L), oder eine Art von Durchschnittswert oder ein anderer Restspannungswert, berechnet aus dem Restspannungsprofil σ(x), ist, Präsentieren der Härtungsgrad-Referenzvariable an einem Anzeigeterminal (12), und/oder Vergleichen der ausgewählten Referenzvariable mit einem vorbestimmten Schwellenwert, und unter der Bedingung, dass der Vergleich zwischen der Referenzvariable und dem Schwellenwert ein vorbestimmtes Kriterium erfüllt, Erzeugen eines Alarms und/oder Erfassen, dass die Glasscheibe nicht als Sicherheitsglas geeignet ist, Präsentieren des Alarms und/oder einer Information zum Erfassen, dass die Glasscheibe nicht als Sicherheitsglas geeignet ist, an einem Anzeigeterminal (12).

2. Verfahren nach Anspruch 1, **dadurch gekennzeichnet, dass** das vorbestimmte Kriterium eine Abweichung zwischen der Referenzvariable und dem Schwellenwert ist.

3. Verfahren nach Anspruch 1, **dadurch gekennzeichnet, dass** die Temperatur des Härteofens (1) und/oder die Erwärmungszeit neu angepasst werden, wenn das vorbestimmte Kriterium erfüllt ist.

4. Verfahren nach Anspruch 1, **dadurch gekennzeichnet, dass** der Blasdruck und/oder der Blasabstand der Luftdüsen der Härtungskühleinheit (2) neu angepasst werden, wenn das vorbestimmte Kriterium erfüllt ist.

5. Verfahren nach Anspruch 1, **dadurch gekennzeichnet, dass** die Referenzvariable proportional zur Partikelanzahl der Glasscheibe ist.

6. Verfahren nach Anspruch 1, **dadurch gekennzeichnet, dass** die Härtungsgrad-Referenzvariable das berechnete Restspannungsprofil an der Mittendicke der Glasscheibe, σ(0,5L), oder eine Partikelanzahl N_{cal} der Glasscheibe, die auf dieser Basis berechnet wird, ist.

7. Verfahren nach Anspruch 1, **dadurch gekennzeichnet, dass** die erreichte Temperatur der Glasbeschickung über die gesamte Oberfläche der Glascharge gemessen wird und aus dem gemessenen Temperaturfeld (T_{i,y-z}) der Durchschnitt aller Messpunkte innerhalb des Temperaturfeldes als gemessene Härtungstemperatur (Tₘₑₐ) der Glasscheiben einer Glasbeschickung ausgewählt wird.

8. Verfahren nach Anspruch 1, **dadurch gekennzeichnet, dass** die erreichte Temperatur der Glasscheibe über die gesamte Oberfläche der Glasscheibe gemessen wird und aus dem gemessenen Temperaturfeld (T_{i,y-z}) der Durchschnitt von Messpunkten innerhalb des Temperaturfeldes oder eines Teilabschnitts davon als gemessene Härtungstemperatur (Tₘₑₐ) der Glasscheibe ausgewählt wird.

9. Verfahren nach Anspruch 1, **dadurch gekennzeichnet, dass** die Flächen der Glasscheibe mit Luftdüsen gekühlt werden, deren konvektiven Kühlleistungen (q_{c,u} and q_{c,l}) aus der Gleichung q_{c,u} = hᵤ[Tₐᵢᵣ-T(0,t)] und q_{c,l} = hₗ[Tₐᵢᵣ-T(L,t)] berechnet werden.

10. Verfahren nach Anspruch 9, **dadurch gekennzeichnet, dass** die gemessenen Blasdrücke (pᵤ, pₗ) als Ausgangsdaten für die Berechnung des konvektiven Wärmeübergangskoeffizienten (hᵤ, hₗ) verwendet werden.

11. Verfahren nach Anspruch 9, **dadurch gekennzeichnet, dass** die gemessenen Blasabstände (Hᵤ, Hₗ) als Ausgangsdaten für die Berechnung des konvektiven Wärmeübergangskoeffizienten (hᵤ, hₗ) verwendet werden.

12. Verfahren nach Anspruch 9, **dadurch gekennzeichnet, dass** die gemessene Luftdüsentemperatur Tₐᵢᵣ zur Berechnung der konvektiven Kühlleistung der Glasscheibenfläche verwendet wird.

13. Verfahren nach Anspruch 1, **dadurch gekennzeichnet, dass** die erreichte Temperatur der Glasscheibe an der Unterseite der Glasscheibe gemessen wird.

## Revendications

1. Procédé de trempe d'une feuille de verre avec un appareil de trempe (20) et de surveillance du niveau de trempe d'une feuille de verre trempée pendant la production, lequel procédé comprend :
- l'identification de propriétés de la feuille de verre, lesdites propriétés incluant de préférence l'épaisseur (L) de la feuille de verre,
- sur la base des propriétés identifiées, l'ajustement de la température du four de trempe (1) et/ou du temps de chauffage de la feuille de verre (G), l'ajustement de la pression de soufflage et/ou de la distance de soufflage des jets d'air de l'unité de refroidissement de trempe (2),
- le chauffage de la feuille de verre dans un four de trempe,
- la mesure de la température atteinte par la feuille de verre à la surface de la feuille de verre après le four de trempe et avant l'unité de refroidissement,
- le refroidissement de la feuille de verre dans une unité de refroidissement de trempe,
**caractérisé en ce que** le procédé comprend, dans l'unité de calcul (10) reliée à l'appareil de trempe (20) :
- le calcul, sur la base de la température mesurée de la feuille de verre, de l'évolution du profil de température dans la direction de l'épaisseur de la feuille de verre pendant le refroidissement de trempe, et le calcul, sur la base de l'évolution calculée du profil de température dans la direction de l'épaisseur, du profil de contraintes résiduelles dans l'épaisseur obtenu dans la feuille de verre pendant la trempe,
- la sélection, sur la base du profil calculé de contraintes résiduelles dans l'épaisseur, d'une variable de référence du niveau de trempe de la feuille de verre, la variable de référence du niveau de trempe de la feuille de verre étant de préférence la contrainte résiduelle à mi-épaisseur du verre, σ(0,5L), ou la contrainte résiduelle à la surface du verre, σ(0) ou σ(L), ou un type de valeur moyenne ou une autre valeur de contrainte résiduelle calculée à partir du profil de contraintes résiduelles σ(x),
la présentation de la variable de référence du niveau de trempe sur un terminal d'affichage (12), et/ou la comparaison de la variable de référence sélectionnée à une valeur seuil prédéterminée et, à condition que la comparaison entre la variable de référence et la valeur seuil satisfasse à un critère prédéterminé, la création d'une alarme et/ou la détermination que la feuille de verre ne peut pas être considérée comme un verre de sécurité, la présentation, sur un terminal d'affichage (12), de l'alarme et/ou d'informations indiquant que la feuille de verre ne peut pas être considérée comme un verre de sécurité.

2. Procédé selon la revendication 1, **caractérisé en ce que** le critère prédéterminé est un écart entre la variable de référence et la valeur seuil.

3. Procédé selon la revendication 1, **caractérisé en ce que** la température du four de trempe (1) et/ou le temps de chauffage sont réajustés lorsque le critère prédéterminé est satisfait.

4. Procédé selon la revendication 1, **caractérisé en ce que** la pression de soufflage et/ou la distance de soufflage des jets d'air de l'unité de refroidissement de trempe (2) sont réajustées lorsque le critère prédéterminé est satisfait.

5. Procédé selon la revendication 1, **caractérisé en ce que** la variable de référence est proportionnelle au nombre de particules de la feuille de verre.

6. Procédé selon la revendication 1, **caractérisé en ce que** la variable de référence du niveau de trempe est le profil de contrainte résiduelle calculé à mi-épaisseur de la feuille de verre, σ(0,5L), ou un nombre de particules N_{cal} de la feuille de verre calculé sur la base de celui-ci.

7. Procédé selon la revendication 1, **caractérisé en ce que** la température atteinte par le chargement de verre est mesurée sur toute la superficie du chargement de verre et, dans le champ de température mesuré (T_{i,y,z}), la moyenne de tous les points de mesure situés dans le champ de température est sélectionnée comme température de trempe mesurée (Tₘₑₐ) des feuilles de verre d'un chargement de verre.

8. Procédé selon la revendication 1, **caractérisé en ce que** la température atteinte par la feuille de verre est mesurée sur toute la superficie de la feuille de verre et, dans le champ de température mesuré (T_{i,y,z}), la moyenne des points de mesure situés dans le champ de température ou dans une sous-section de celui-ci est sélectionnée comme température de trempe mesurée (Tₘₑₐ) de la feuille de verre.

9. Procédé selon la revendication 1, **caractérisé en ce que** les surfaces de la feuille de verre sont refroidies avec des jets d'air dont les puissances de refroidissement convectif (q_{c,u} et q_{c,l}) sont calculées à partir des équations q_{c,u} = hᵤ[Tₐᵢᵣ - T(0,t)] et q_{c,l} = hl[Tₐᵢᵣ - T(L,t)].

10. Procédé selon la revendication 9, **caractérisé en ce que** les pressions de soufflage mesurées (pᵤ, pₗ) sont utilisées comme données initiales pour calculer les coefficients de transfert thermique convectif (hᵤ, hₗ).

11. Procédé selon la revendication 9, **caractérisé en ce que** les distances de soufflage mesurées (Hᵤ, Hₗ) sont utilisées comme données initiales pour calculer les coefficients de transfert thermique convectif (hᵤ, hₗ).

12. Procédé selon la revendication 9, **caractérisé en ce que** la température mesurée Tₐᵢᵣ des jets d'air est utilisée pour calculer la puissance de refroidissement convectif de la surface de la feuille de verre.

13. Procédé selon la revendication 1, **caractérisé en ce que** la température atteinte par la feuille de verre est mesurée sur la surface inférieure de la feuille de verre.
